# EUROPEAN PATENT SPECIFICATION

(11) **EP 2 209 802 B1**
(45) Date of publication and mention of the grant of the patent: **21.10.2015**
(21) Application number: 08833608.6
(22) Date of filing: 19.09.2008
(51) Int. Cl.: C07K 16/00, C07H 21/04, C07K 16/26

(54) **RESISTIN ANTAGONISTS AND THEIR USE**
RESISTINANTAGONISTEN UND IHRE VERWENDUNG
ANTAGONISTES DE RÉSISTINE ET LEUR UTILISATION

(30) Priority: 24.09.2007 US 974607 P
(43) Date of publication of application: 28.07.2010
(73) Proprietor: Janssen Biotech, Inc., Horsham, PA 19044 (US)
(72) Inventor: CARTON, Jill, Radnor, PA 19087 (US); FARRELL, Francis, Radnor, PA 19087 (US); MURRAY, Lynne, Narberth, PA 19072 (US); ORT, Tatiana, Radnor, PA 19087 (US); TEPLYAKOV, Alexey, Radnor, PA 19087 (US)
(74) Representative: Goodfellow, Hugh Robin
(86) International application number: PCT/US2008/076934
(87) International publication number: WO 2009/042504

(56) References cited:
- WO-A2-03/085093
- WO-A2-2008/052111
- PANG ET AL.: 'Role of resistin in inflammation and inflammation-related diseases.' CELLULAR AND MOLECULAR IMMUNOLOGY. vol. 3, no. 1, February 2006, pages 29 - 34, XP003026577
- BERTOLANI ET AL.: 'Resistin as an intrahepatic cytokine.' AMERICAN JOURNAL OF PATHOLOGY. vol. 169, no. 6, December 2006, pages 2042 - 2053, XP003026578

### SEQUENCE LISTING

<110> CENTOCOR, INC.
<120> RESISTIN ANTAGONISTS AND THEIR USE
<130> CEN5194PCT
<140> PCT/US2008/076934
   <141> 2008-09-19
<150> 60/974,607
   <151> 2007-09-24
<160> 6
<170> FastSEQ for Windows Version 4.0
<210> 1
   <211> 108
   <212> PRT
   <213> Homo sapiens
<400> 1
   Met Lys Ala Leu Cys Leu Leu Leu Leu Pro Val Leu Gly Leu Leu Val
    1               5                  10                  15
   Ser Ser Lys Thr Leu Cys Ser Met Glu Glu Ala Ile Asn Glu Arg Ile
               20                  25                  30
   Gln Glu Val Ala Gly Ser Leu Ile Phe Arg Ala Ile Ser Ser Ile Gly
           35                  40                  45
   Leu Glu Cys Gln Ser Val Thr Ser Arg Gly Asp Leu Ala Thr Cys Pro
       50                  55                  60
   Arg Gly Phe Ala Val Thr Gly Cys Thr Cys Gly Ser Ala Cys Gly Ser
   65                  70                  75                  80
   Trp Asp Val Arg Ala Glu Thr Thr Cys His Cys Gln Cys Ala Gly Met
                   85                  90                  95
   Asp Trp Thr Gly Ala Arg Cys Cys Arg Val Gln Pro
               100                 105
<210> 2
   <211> 114
   <212> PRT
   <213> Mus musculus
<400> 2
   Met Lys Asn Leu Ser Phe Pro Leu Leu Phe Leu Phe Phe Leu Val Pro
    1              5                  10                  15
   Glu Leu Leu Gly Ser Ser Met Pro Leu Cys Pro Ile Asp Glu Ala Ile
               20                  25                  30
   Asp Lys Lys Ile Lys Gln Asp Phe Asn Ser Leu Phe Pro Asn Ala Ile
           35                  40                  45
   Lys Asn Ile Gly Leu Asn Cys Trp Thr Val Ser Ser Arg Gly Lys Leu
       50                  55                  60
   Ala Ser Cys Pro Glu Gly Thr Ala Val Leu Ser Cys Ser Cys Gly Ser
   65                  70                  75                  80
   Ala Cys Gly Ser Trp Asp Ile Arg Glu Glu Lys Val Cys His Cys Gln
                   85                  90                  95
   Cys Ala Arg Ile Asp Trp Thr Ala Ala Arg Cys Cys Lys Leu Gln Val
               100                 105                 110
   Ala Ser
<210> 3
   <211> 16
   <212> PRT
   <213> Homo sapiens
<400> 3
   Glu Cys Gln Ser Val Thr Ser Arg Gly Asp Leu Ala Thr Cys Pro Arg
    1              5                  10                  15
<210> 4
   <211> 16
   <212> PRT
   <213> Homo sapiens
<400> 4
   Cys Gly Ser Trp Asp Val Arg Ala Glu Thr Thr Cys His Cys Gln Cys
    1              5                  10                  15
<210> 5
   <211> 16
   <212> PRT
   <213> Homo sapiens
<400> 5
   Glu Ser Gln Ser Val Thr Ser Arg Gly Asp Leu Ala Thr Ser Pro Arg
    1              5                  10                  15
<210> 6
   <211> 16
   <212> PRT
   <213> Homo sapiens
<400> 6
   Ser Gly Ser Trp Asp Val Arg Ala Glu Thr Thr Ser His Ser Gln Ser
    1              5                  10                  15

## Description

### Field of the Invention

The present invention relates to resistin antagonists such as antibodies and their use in treating diseases with fibrotic pathologies and antigens useful for generating anti-resistin antibodies.

### Background of the Invention

Interstitial lung disease (ILD) is a collective term for more than 100 different diseases with similar clinical, radiological and physiological characteristics. The underlying causes can be due to systemic illness or occupational exposure. However, some ILD have unknown underlying etiology. These fibrotic lung diseases are difficult to treat, with biopsies being required for diagnosis.

The most common fibrotic lung disease is UIP (usual interstitial pneumonia) with an incidence of between 3-29 per 100,000 (Coultas et al., Am. J. Respir. Crit. Care Med. 150:967-72 (1994)). UIP develops insidiously over a few months to several years and often radiological changes appear before clinical symptoms. Characteristic histological findings of UIP include the presence of patchy, heterogenous lung fibrosis, which, as the disease progresses can result in alveolar collapse, bronchiolectasis and honeycombing. Nonspecific interstitial pneumonia (NSIP) presents with similar clinical symptoms to UIP. However, although certain histological findings are similar, this patient group tends to have less fibrosis (MacDonald et al., Radiology 221:600-5 (2001)). Furthermore, NSIP patients are more responsive to corticosteroids and in one study, NSIP patients had considerably improved prognosis (Daniil et al., Am. J. Respir. Crit. Care Med. 160:899-905 (1999)).

There have been reports in the literature that there are differences in fibroblasts obtained from fibrotic lung disease compared to cells obtained from non-fibrosis related lung disease, such as samples isolated from the normal margins of lung tumor resections. Tissue sections from UIP patients express increased levels of CXCL8 (also known as Interleukin 8 or IL8), an angiogenic chemokine, and decreased levels of an angiostatic chemokine CXCL10 (IFN-inducible Protein of 10kDa, IP10), thereby suggesting an imbalance in net angiogenesis in the lungs of UIP patients (Keane et al., J. Immunol. 159:1437-43 (1997)). Moreover, fibroblasts from these patients also expressed increased CXCL8 suggesting that the fibroblast is the main effector cell causing the angiogenic imbalance (Keane et *al., supra*). More recently it has been shown that fibroblasts from UIP patients express and secrete increased levels of CCL7 (Monocyte Chemotactic Protein 3, MCP3) (Choi et al., Am. J. Respir. Crit. Care Med. 170:508-15 (2004)). Studies comparing TGFβ1-induced gene expression in fibroblasts from non-fibrotic tissue, UIP and scleroderma-induced lung fibrosis, have also demonstrated distinct phenotypes and responses of fibroblasts depending on the environment the cells have been isolated from (Renzoni et al., Respir. Res. 5:24 (2004)).

Resistin is a secreted factor that belongs to the FIZZ (Found in Inflammatory Zone, also known as RELM) protein family containing a conserved C-terminal Cys-rich domain (Steppan et al., Proc. Natl. Acad. Sci. U.S.A. 98:502-506 (2001)). It was originally described in mice as an adipocyte-derived polypeptide that provided the link between obesity and insulin resistance (Steppan et al., Nature 409:307-312 (2001)). Human resistin, on the other hand, is a macrophage/monocyte-derived factor recognized as a potent proinflammatory factor. Human resistin induces cytokine release from various cells of immune origin, up-regulates the expression of adhesion molecules and promotes angiogenesis in endothelial cells (Burnett et al., Atherosclerosis 182:241-8 (2005); Mu et al., Cardiovasc. Res. 70:146-57 (2006); Verma et al., Circulation 108:736-40 (2003); Bokarewa et al., J. Immunol. 174:5789-95 (2005)). The effect of resistin is likely mediated through the NF-κb pathway (Bokarewa *et al., supra*).

Currently four members of the FIZZ family have been identified in rodents and two in humans. Among them FIZZ1 (RELMα) is implicated in the development of lung fibrosis in mice. FIZZ1 was discovered in lavage fluid in a murine allergic pulmonary model (Holcomb et al., EMBO J. 19:4046-55 (2000)). In addition, up-regulation of the FIZZ1 gene was found in lung from bleomycin-induced fibrotic mice (Liu et al., Am. J. Pathol. 164):1315-26 (2004)). Intravenous injection of FIZZ1 into mice showed marked increase of CD68-positive macrophages in the lungs (Yamaji-Kegan et al., J. Physiol. Lung Cell. Mol. Physiol. August 4, 2006 e-print). In vitro studies showed that FIZZ1 has mitogenic properties and may mediate fibrosis through the stimulation of smooth muscle cell proliferation and induction of actin and collagen deposition in lung fibroblasts (Teng et al., Circ. Res. 92:1065-7 (2003)). Intratracheal instillation of FIZZ1 resulted in significant increase of VEGF production suggesting the role of FIZZ1 in pulmonary angiogenesis (Tong et al., Respir. Res. 7:37 (2006)). A FIZZ1 orthologue has not been identified in the human genome. Interestingly, human resistin (FIZZ3) shows a greater similarity in expression pattern to murine FIZZ1 then murine resistin suggesting a potential functional similarity. However, the relationship between human resistin and lung fibrosis remains largely unexplored.

A resistin receptor has not been identified, which limits the understanding of resistin biology. Recently, the three-dimensional structure of mouse resistin has been solved (Patel et al., Science 304:1154-8 (2004). Mouse resistin forms noncovalent trimeric oligomers that may aggregate into disulphide-linked hexameric structures. There is no structural information on human resistin.

Thus, in view of the above, a need exists for structural information on human resistin and for therapeutics, such as resistin antagonists, to treat lung fibrosis and its associated symptoms.

WO 03/085093 describes antibodies and related molecules that immunospecifically bind to antigenic GMAD peptides.

### Brief Description of the Drawings

Fig. 1 shows resistin levels in lung biopsies from UIP, NSIP and non-fibrotic patients.
Figs. 2A, B, and C show the effects of resistin on induction of gene expression of procollagen I and α-smooth muscle actin (αSMA) in fibrotic lung and non-fibrotic lung derived fibroblasts and after primary lung fibroblasts isolated from fibrotic lung biopsy were treated with recombinant resistin.
Figs. 3A and B shows the effect of resistin on gene expression of pro-fibrotic growth factors and growth factor receptors in primary lung fibroblasts.
Figs. 4A and B show the induction of expression level of several extracellular matrix related genes (4A) and IL-6 gene (4B) in lung fibroblasts.
Figs. 5A, B, and C show the increase in the release of pro-inflammatory cytokines IL-6 (5A), IL-8 (5B) and MCP-1 (5C) after human lung primary fibroblasts were treated with recombinant resistin.
Fig. 6 shows the secretion of resistin from human primary monocytes. Non-adherent or adherent monocytes were untreated or stimulated with pro-fibrotic modulators, * indicates p-value < 0.05.
Figs. 7A and B show co-localization of resistin with macrophage-like cells in UIP tissue sections.
Fig. 8 shows a human and mouse resistin amino acid sequence alignment.
Fig. 9 shows binding of polyclonal antibodies C2815 and C2816 to full-length human resistin.
Figs. 10A and B show the effect of polyclonal antibodies C2815 and C2816 on human resistin-mediated MCP-1 release from isolated human primary blood mononuclear cells (PBMC).
Figs. 11A and B show the effect of the C2815 and C2816 polyclonal antibodies on mouse resistin-mediated MCP-1 release from isolated human PBMC.

### Summary of the Invention

The invention relates to an antagonist of resistin such as an isolated antibody reactive with resistin which neutralizes at least one of its biological activities.

One aspect of the invention is an isolated antibody reactive with an epitope located at residues 78 to 93 (SEQ ID NO: 4) of human resistin.

Another aspect of the invention is a pharmaceutical composition comprising an effective amount of the resistin antagonist of the invention and a pharmaceutically acceptable carrier or diluent.

Another aspect of the invention is the pharmaceutical composition of the invention for use in a method of modifying the biological activity of resistin in a mammal comprising administering the pharmaceutical composition to the mammal. Another aspect of the invention is the pharmaceutical composition of the invention for use in a method of treating, or alleviating the symptoms of the diseases with aberrant fibroblast activity including interstitial lung diseases, hypertrophic scarring, keloid scarring, scleroderma, liver fibrosis, renal fibrosis and cardiac fibrosis comprising administering the pharmaceutical composition to a patient in need thereof.

### Detailed Description of the Invention

This invention relates to the role of resistin, a cytokine, as a novel therapeutic target for resistin antagonists to treat diseases associated with fibrotic pathologies such as ILD, scleroderma, hypertrophic scarring, as well as diseases associated with significant remodeling in the lung such as asthma and chronic obstructive pulmonary disorder (COPD).

In the present disclosure, it has been shown that resistin protein is detected in fibrotic lungs. Immunohistochemistry with anti-resistin monoclonal antibodies indicate that resistin is expressed in monocyte/macrophage cells that are involved in fibrotic and remodeling processes. Resistin secretion is induced by pro-fibrotic modulators suggesting that elevated local levels of resistin might be present in fibrotic lung. Resistin directly affects primary lung fibroblasts by inducing genes associated with extracellular matrix (ECM) accumulation and myofibroblast differentiation, two processes that contribute to pathogenesis of lung fibrosis. Resistin also induces the expression of pro-fibrotic growth factors and their receptors in lung fibroblasts. Furthermore, resistin stimulates inflammatory response in lung fibroblasts by increasing the release of pro-inflammatory cytokines. Resistin affects fibrotic cells more then non-fibrotic fibroblasts suggesting that resistin signaling is up-regulated in diseased fibroblasts. Finally, in the present disclosure it is shown that resistin expression and secretion are induced by pro-fibrotic modulators. Taken together, these data suggest that resistin is able to exacerbate fibrosis by contributing to fibroblast activation and lung inflammation. Accordingly, inhibition of resistin will be beneficial for the treatment of diseases associated with aberrant fibroblast function.

Further, the present disclosure describes a model of the three-dimensional structure of human resistin that was based on the crystal structure of the mouse protein. This model is used to predict a potential receptor-binding site based on structural similarity to the C1q/TNF superfamily. Two polyclonal antibodies have been raised against peptides spanning the predicted receptor-binding site and the antibodies neutralizing activity was confirmed in *in vitro* functional assay. The data suggest that the identified epitopes are important for resistin function and can be used for generation of therapeutics agents neutralizing resistin.

In an embodiment of the disclosure, the epitope peptides are conjugated to a carrier protein or fused to a larger protein scaffold for use as an antigen for immunization and/or for phage display panning for generation of neutralizing anti-resistin antibody. The disclosed functional epitopes can be used for screening a panel of antibodies to identify those with neutralizing activity.

### Antibody Polypeptides and Compositions

The present invention provides an antagonist against resistin. In one embodiment, the resistin antagonist is an antibody having the properties of binding resistin and neutralizing at least one resistin biological activity.

The term "antagonist" is used in the broadest sense and includes a molecule that is capable of, directly or indirectly, partially or fully counteracting, reducing or inhibiting one or more biological activities of resistin.

The term "antibody" is used in the broadest sense and includes immunoglobulin or antibody molecules including polyclonal antibodies, monoclonal antibodies including murine, human, humanized and chimeric monoclonal antibodies and antibody fragments.

In general, antibodies are proteins or polypeptides that exhibit binding specificity to a specific antigen. Intact antibodies are heterotetrameric glycoproteins, composed of two identical light chains and two identical heavy chains. Typically, each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain and the light chain variable domain is aligned with the variable domain of the heavy chain. Antibody light chains of any vertebrate species can be assigned to one of two clearly distinct types, namely kappa (κ) and lambda (λ), based on the amino acid sequences of their constant domains. Immunoglobulins can be assigned to five major classes, namely IgA, IgD, IgE, IgG and IgM, depending on the heavy chain constant domain amino acid sequence. IgA and IgG are further sub-classified as the isotypes IgA1, IgA2, IgG1, IgG2, IgG3 and IgG4.

The term "antibody fragments" means a portion of an intact antibody, generally the antigen binding or variable region of the intact antibody. Examples of antibody fragments include Fab, Fab', F(ab')2 and Fv fragments, and single chain antibody molecules.

"CDRs" are defined as the complementarity determining region amino acid sequences of an antibody which are the hypervariable regions of immunoglobulin heavy and light chains. See, e.g., Kabat et al., Sequences of Proteins of Immunological Interest, 4th ed., U.S. Department of Health and Human Services, National Institutes of Health (1987). There are three heavy chain and three light chain CDRs or CDR regions in the variable portion of an immunoglobulin. Thus, "CDRs" as used herein refers to all three heavy chain CDRs, or all three light chain CDRs or both all heavy and all light chain CDRs, if appropriate.

CDRs provide the majority of contact residues for the binding of the antibody to the antigen or epitope. CDRs of interest in this invention are derived from donor antibody variable heavy and light chain sequences, and include analogs of the naturally occurring CDRs, which analogs also share or retain the same antigen binding specificity and/or neutralizing ability as the donor antibody from which they were derived.

The term "monoclonal antibody" (mAb) as used herein means an antibody (or antibody fragment) obtained from a population of substantially homogeneous antibodies. Monoclonal antibodies are highly specific, typically being directed against a single antigenic determinant. The modifier "monoclonal" indicates the substantially homogeneous character of the antibody and does not require production of the antibody by any particular method. For example, murine mAbs can be made by the hybridoma method of Kohler et al., Nature 256:495-497 (1975). Chimeric mAbs containing a light chain and heavy chain variable region derived from a donor antibody (typically murine) in association with light and heavy chain constant regions derived from an acceptor antibody (typically another mammalian species such as human) can be prepared by the method disclosed in U.S. Pat. No. 4,816,567. Humanized mAbs having CDRs derived from a non-human donor immunoglobulin (typically murine) and the remaining immunoglobulin-derived parts of the molecule being derived from one or more human immunoglobulins, optionally having altered framework support residues to preserve binding affinity, can be obtained by the techniques disclosed in Queen et al., Proc. Natl. Acad. Sci. (USA), 86:10029-10032 (1989) and Hodgson et al., Bio/Technology, 9:421 (1991). Exemplary human framework sequences useful for humanization are disclosed at, e.g., www.ncbi.nlm.nih.gov/entrez/query.fcgi; www.ncbi.nih.gov/igblast; www.atcc.org/phage/hdb.html; www.mrc-cpe.cam.ac.uk/ALIGNMENTS.php; www.kabatdatabase.com/top.html; ftp.ncbi.nih.gov/repository/kabat; www.sciquest.com; www.abcam.com; www.antibodyresource.com/onlinecomp.html; www.public.iastate.edu/~pedro/research_tools.html; www.whfreeman.com/immunology/CH05/kuby05.htm; www.hhmi.org/grants/lectures/1996/vlab; www.path.cam.ac.uk/∼mrc7/mikeimages.html; mcb.harvard.edu/BioLinks/Immunology.html; www.immunologylink.com; pathbox.wustl.edu/∼hcenter/index.html; www.appliedbiosystems.com; www.nal.usda.gov/awic/pubs/antibody; www.m.ehime-u.ac.jp/∼yasuhito/Elisa.html; www.biodesign.com; www.cancerresearchuk.org; www.biotech.ufl.edu; www.isac-net.org; baserv.uci.kun.nl/∼jraats/links1.html; www.recab.uni-hd.de/immuno.bme.nwu.edu; www.mrc-cpe.cam.ac.uk; www.ibt.unam.mx/vir/V_mice.html; http://www.bioinf.org.uk/abs; antibody.bath.ac.uk; www.unizh.ch; www.cryst.bbk.ac.uk/∼ubcg07s; www.nimr.mrc.ac.uk/CC/ccaewg/ccaewg.html; www.path.cam.ac.uk/∼mrc7/humanisation/TAHHP.html; www.ibt.unam.mx/vir/structure/stat aim.html; www.biosci.missouri.edu/smithgp/index.html; www.jerini.de; imgt.cines.fr; and Kabat et al., Sequences of Proteins of Immunological Interest, U.S. Dept. Health (1987).

Fully human mAbs lacking any non-human sequences can be prepared from human immunoglobulin transgenic mice by techniques referenced in, e.g., Lonberg et al., Nature 368:856-859 (1994); Fishwild et al., Nature Biotechnology 14:845-851 (1996) and Mendez et al., Nature Genetics 15:146-156 (1997). Human mAbs can also be prepared and optimized from phage display libraries by techniques referenced in, e.g., Knappik et al., J. Mol. Biol. 296:57-86 (2000) and Krebs et al., J. Immunol. Meth. 254:67-84 (2001).

The present invention relates to resistin antagonists capable of binding human resistin and modulating NF-κb mediated signaling. The present invention also relates to resistin antagonists capable of binding human resistin and modulating resistin-mediated activities including, but not limited to, aberrant activation of fibroblasts, induction pro-fibrotic gene expression, stimulation of inflammatory responses. Such antagonists include anti-resistin antibodies having the properties of binding resistin and modulating resistin signaling. One aspect of the invention is an antibody reactive with human resistin that inhibits resistin-mediated release of other inflammatory cytokines including, but not limited to MCP-1, IL-8 and IL-6. These antibodies are useful as research reagents, diagnostic reagents and therapeutic agents. In particular, the antibodies of the invention are useful as therapeutic agents for treating fibrosis or alleviating symptoms of fibrosis.

Exemplary antibodies may be antibodies of the IgG, IgD, IgGA or IgM isotypes. Additionally, such antibodies can be post-translationally modified by processes such as glycosylation, isomerization, aglycosylation or non-naturally occurring covalent modification such as the addition of polyethylene glycol moieties (pegylation) and lipidation. Such modifications may occur *in vivo* or *in vitro.* Fully human, humanized and affinity-matured antibody molecules or antibody fragments are within the scope of the invention as are mimetibodies, fusion proteins and chimeric proteins.

The antibodies of the invention may bind to the human resistin epitope ₇₈CGSWDVRAETTCHCQC₉₃ (SEQ ID NO: 4). This resistin epitope can be isolated and modified for use as an antigen to produce and screen monoclonal antibodies against human resistin. For example, the epitope sequence can be modified to remove the cysteine residues to prevent disulfide bonding. These residues can be replaced with serine.

The antibodies of the invention may bind human resistin with a K_{d} less than or equal to about 10⁻⁷, 10⁻⁸, 10⁻⁹, 10⁻¹⁰, 10⁻¹¹ or 10⁻¹² M. The affinity of a given antibody against human resistin can be determined experimentally using any suitable method. Such methods may utilize Biacore or KinExA instrumentation, ELISA or competitive binding assays known to those skilled in the art. Antibody molecules binding human resistin with a desired affinity can be selected from libraries of variants or fragments by techniques including antibody affinity maturation and other suitable art-recognized techniques.

Another aspect of the present invention is pharmaceutical compositions comprising at least one resistin antibody and a pharmaceutically acceptable carrier or diluent known in the art. The carrier or diluent can be a solution, suspension, emulsion, colloid or powder.

A resistin antibody of the invention is formulated as a pharmaceutical composition in a therapeutically effective amount. The term "effective amount" generally refers to the quantities of antibody necessary for effective therapy, i.e., the partial or complete alleviation of the symptom or disorder for which treatment was sought. Included within the definition of effective therapy are prophylactic treatments intended to reduce the likelihood of onset of the above-described symptoms or disorders.

### Nucleic acids, vectors and cell lines

Another aspect of the present disclosure is isolated nucleic acid molecules comprising, complementary to or having significant identity with a polynucleotide encoding an antibody heavy or light chain having CDR amino acid sequences. Other polynucleotides which, given the degeneracy of the genetic code or codon preferences in a given expression system, encode the heavy or light chain variable region CDRs are also within the scope of the disclosure.

Typically, the nucleic acids of the present disclosure are used in expression vectors for the preparation of the resistin antibody polypeptides of the invention. Vectors within the scope of the disclosure provide necessary elements for eukaryotic expression, including viral promoter driven vectors, such as CMV promoter driven vectors, e.g., pcDNA3.1, pCEP4 and their derivatives, Baculovirus expression vectors, Drosophila expression vectors and expression vectors that are driven by mammalian gene promoters, such as human Ig gene promoters. Other examples include prokaryotic expression vectors, such as T7 promoter driven vectors, *e.g.*, pET41, lactose promoter driven vectors and arabinose gene promoter driven vectors.

The present disclosure also relates to cell lines expressing resistin antibodies. The host cells can be prokaryotic or eukaryotic cells. Exemplary eukaryotic cells are mammalian cells, such as but not limited to, COS-1, COS-7, HEK293, BHK21, CHO, BSC-1, HepG2, 653, SP2/0, NS0, 293, HeLa, myeloma, lymphoma cells, or any derivative thereof. Most preferably, the host cells are HEK293, NS0, SP2/0 or CHO cells. The cell lines may be generated by stable or transient transfection procedures that are well known in the art.

The present disclosure further provides methods for expressing a resistin antibody comprising culturing the cell lines under conditions wherein the resistin antibody is expressed in detectable or recoverable amounts. The present disclosure also provides methods for generating resistin antibody comprising translating the resistin antibody encoding nucleic acids under conditions *in vitro* or *in situ*, such that the resistin antibody is expressed in detectable or recoverable amounts. The present disclosure also encompasses a resistin antibody produced by the above methods.

A resistin antibody can be recovered and purified by well-known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylatpatite chromatography and lectin chromatography. High performance liquid chroatography (HPLC) can also be employed for purification.

### Therapeutic Methods for Treating or Alleviating Symptoms of Fibrosis

The resistin antibodies are useful as, *inter alia*, research reagents and therapeutic agents. In one aspect, the present invention relates to a method of modifying the biological activities of resistin comprising providing resistin antibody of the present invention to a mammal in need thereof. Examples of such biological activities of resistin include aberrant activation of fibroblasts, stimulation of myoblast differentiation, induction of pro-fibrotic genes, release of pro-inflammatory cytokines, induction of collagen I accumulation as well as other activities known to those skilled in the art. Specifically, the resistin antibody may decrease or inhibit resistin-mediated cell signaling cascades, such as but not limited to the NF-κb pathway.

The present invention further provides methods for alleviating the symptoms of, or treating fibrosis comprising administering a therapeutically effective amount of a resistin antibody pharmaceutical composition to a patient in need thereof. As described above, such composition comprises an effective amount of resistin antibody and a pharmaceutically acceptable carrier or diluent. The effective amount for a given therapy, whether curative or preventative, will generally depend upon may different factors, including means of administration, target site and other medicants administered. Thus, treatment doses will need to be titrated to optimize safety and efficacy. In addition, the resistin antibody may be administered singly or in combination with at least one further compound, protein or composition useful for treating fibrosis.

The mode of administration can be any suitable route to deliver the pharmaceutically effective amount of resistin antibody of the present invention to a host. For example, the resistin antibody can be delivered via parenteral administration, such as subcutaneous, intraarcheal, intramuscular, intradermal, intravenous or intranasal administration, or any other means known in the art.

The present invention is further described with reference to the following examples. These examples are merely to illustrate aspects of the present invention and are not intended as limitations of this invention.

### Example 1

### Resistin is Present in Lung

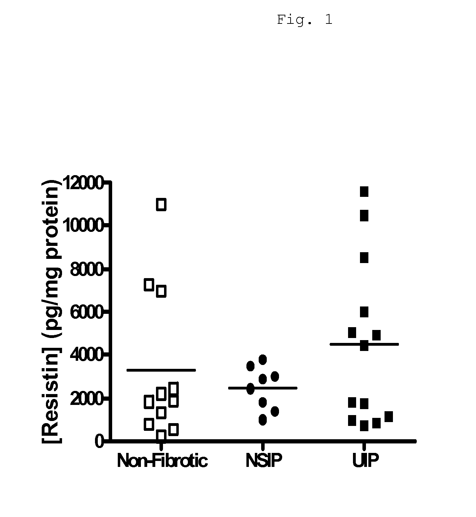

Lung biopsy samples from UIP, NSIP and the normal margins of lung tumors were homogenized in PBS containing Complete Protease Inhibitor (Roche). Resistin levels were measured by ELISA (Linco Research Inc., St. Charles, MO) following the manufacturer's instructions. Total protein was measured in each lung biopsy sample using the BCA protein assay (Pierce Biotechnology, Inc., Rockford, IL). Resistin levels were then normalized to the amount of protein in each patient sample and are shown in Fig. 1. Resistin protein was detected in homogenates derived from all of lung biopsy specimens. No significant difference between the non-fibrotic and fibrotic groups was observed.

### Example 2

### Resistin Stimulates the Expression of Genes Associated with Fibrosis

Pulmonary fibroblasts were isolated from lung biopsies taken from UIP patients (n=4); now referred to herein as "fibrotic fibroblasts" or 'UIP fibroblasts'. Fibroblasts were also isolated from lung tissue taken during lung tumor resection (n=5) and these samples were confirmed to be non-fibrotic by histological analysis. These non-fibrotic tissue derived fibroblasts are referred to herein as "non-fibrotic fibroblasts" (NF).

Human lung fibroblasts were plated into 24 well plates (Costar, Corning, NY) at 100,000 cells/well and allowed to adhere for 8 hours. The cells were then washed with PBS and cultured overnight in serum free media (DMEM with l-Glutamine, Pen/Strep). Cells were then stimulated for 24 hrs in the presence or absence of TGFβ-1 (1 or 10 ng/mL) or resistin (1 or 10 ug/mL). Human recombinant resistin was purchased from Peprotech, Inc (Rocky Hill, NJ). The purity of the protein was confirmed using SDS-PAGE and mass spectroscopy and endotoxin levels were found to be low (0.9 EU/ mg). Supernatants were removed and analyzed for resistin protein using commercially available ELISA or Luminex. RNA was subsequently isolated using RNeasy Plus Mini-Kits (QIAGEN, Valencia, CA) as per manufacturer's instructions and reverse transcribed into cDNA using TaqMan® Reverse Transcription Reagents (Applied Biosystems, Foster City, CA). Profibrotic gene expression was determined by real time PCR using the Taqman® Universal PCR Master Mix (Applied Biosystems) and pre-developed Taqman® Gene Expression Assays (Applied Biosystems) as per manufacturer's instructions. Quantitative gene expression was calculated using the comparative C_{T} method, where C_{T} values are determined as the threshold cycle number for which gene expression is first detected. Fold changes in gene expression for the genes of interest were first normalized to the housekeeping gene 18S, giving ΔC_{T} values. Fold changes in gene expression due to *in vitro* stimulation were calculated by ΔΔC_{T} = ΔC_{T(unstimulated)} - ΔC_{T(stimulated)}, where the unstimulated sample served as calibrator. To investigate potential pro-fibrotic activities of resistin the expression level of procollagen I, alpha smooth muscle actin (αSMA), CTGF, TGFβ1, TGFbR, PDGFR, hyaluronan receptor CD44, fibrillin (fbn1), fibronectin (fn1) and IL-6 were evaluated in untreated and resistin-treated lung fibroblasts.

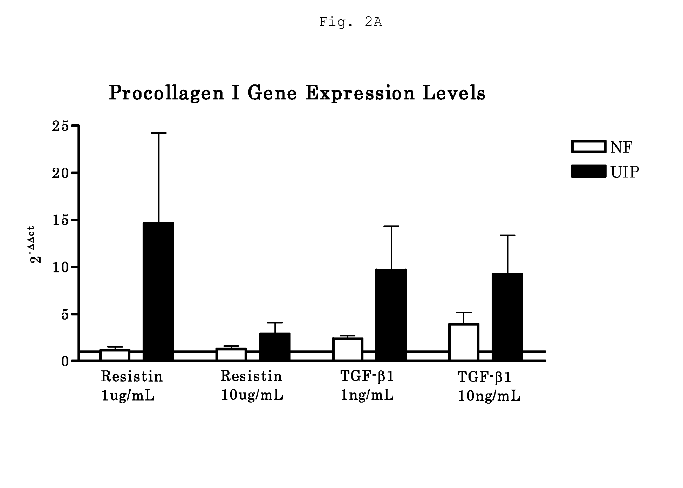

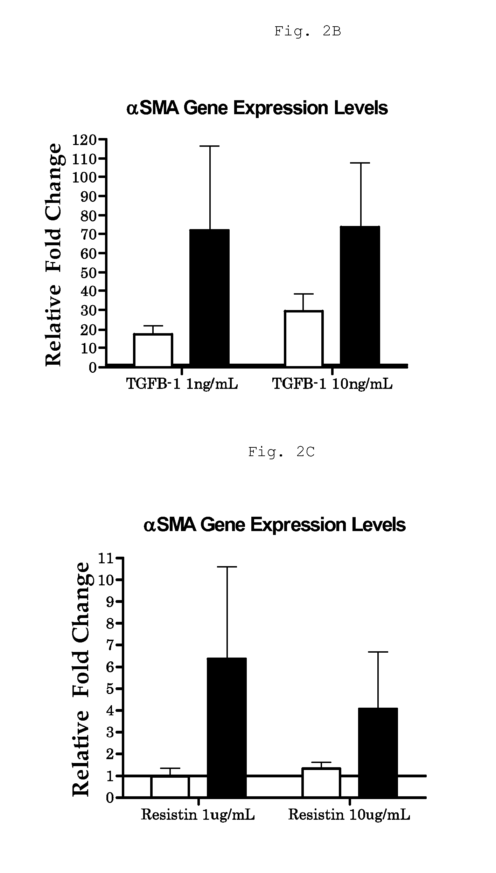

Accumulation of collagen deposition and the presence of myofibroblasts are two key hallmarks of fibrosis. TGFβ1, a known pro-fibrotic modulator used as a positive control in the study, induced an up-regulation of both procollagen I and αSMA in lung fibroblasts (Fig. 2). Similarly, treatment of lung fibroblasts with recombinant resistin resulted in induction of procollagen I and αSMA expression (Fig. 2). Importantly, this resistin effect is specific for fibroblasts isolated from fibrotic patients, as resistin did not affect gene expression in non-fibrotic fibroblasts. These data suggest that resistin receptor/signaling is up-regulated/activated in fibrotic cells.

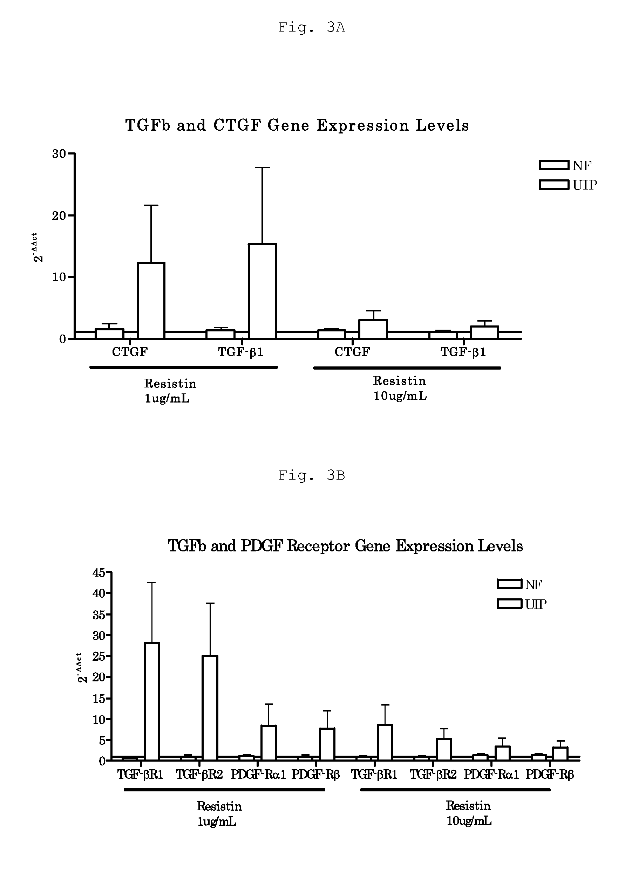

As shown in Fig. 3A, resistin induced an up-regulation of CTGF and TGFβ1 genes, known pro-fibrotic modulators. Furthermore, resistin also induced an up-regulation of TGFβ receptor and PDGF receptor gene expression (Fig. 3B). As with the other genes already described, resistin only augmented gene expression in fibrotic fibroblasts.

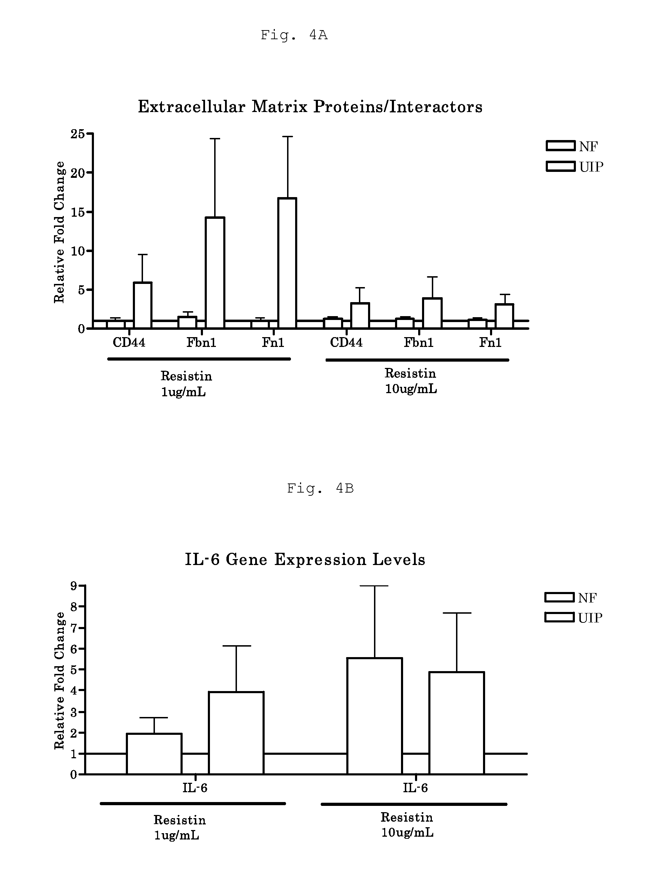

Alterations in extracellular matrix (ECM) components as well as aberrant matrix turnover are hallmarks of various fibrotic diseases or diseases associated with tissue remodeling. The effect of resistin on several of the ECM genes including the hyaluronan receptor CD44, fibrillin (fbn1) and fibronectin (fn1) was evaluated. Resistin enhanced the expression of CD44, fibrillin and fibronectin specifically in fibrotic fibroblasts (Fig. 4A). Resistin-induced gene expression was more pronounced at low concentration (1ug/ml). Resistin also induced an increase in IL-6 expression in both fibrotic and non-fibrotic fibroblasts (Fig. 4B).

### Example 3

### Resistin Promotes a Pro-Inflammatory Environment Through the Induction of Cytokine Release

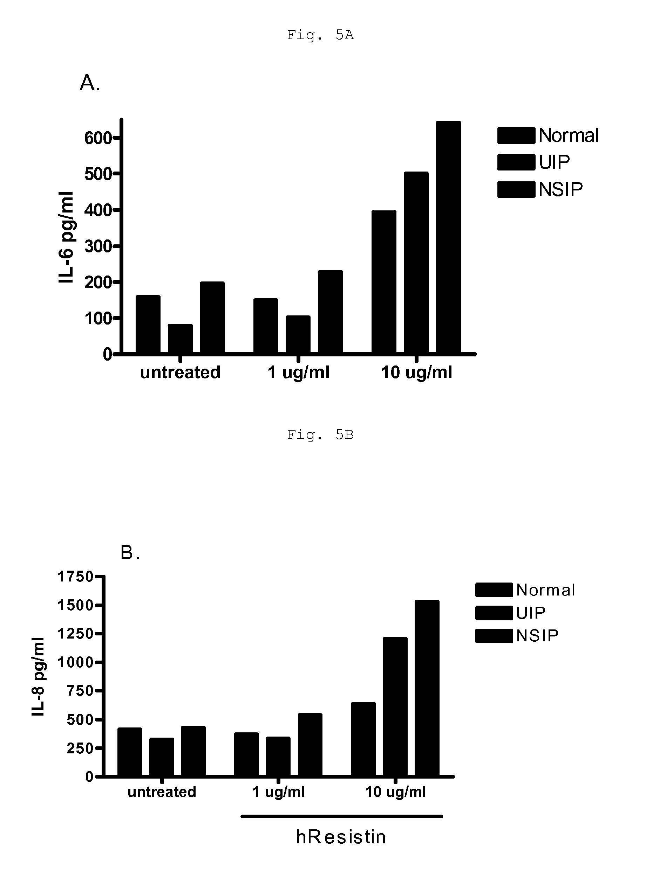

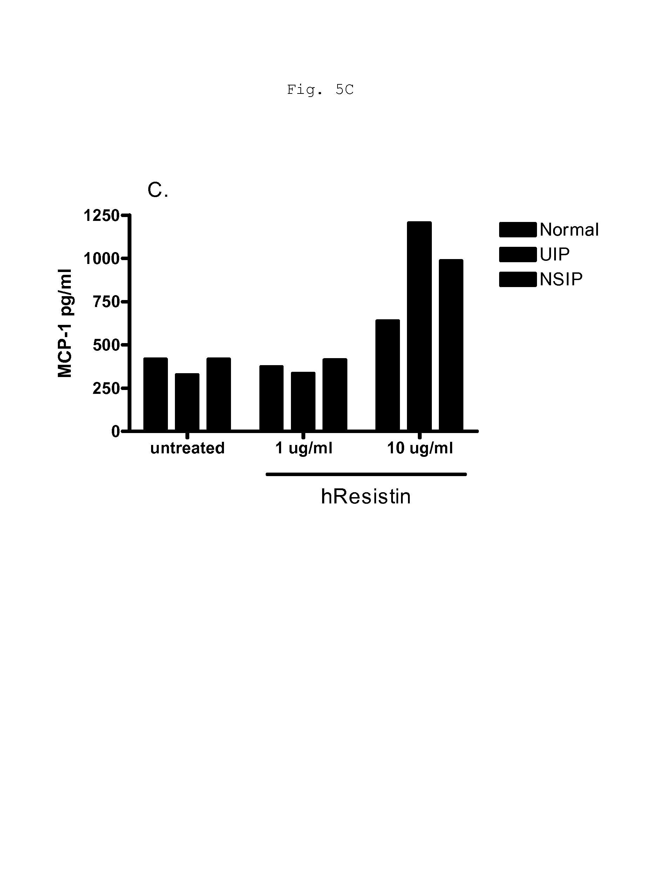

Cell-free supernatants from the fibroblasts isolated from the lung biopsies that were stimulated *in vitro* with human recombinant resistin were analyzed for cytokine levels by the LINCO human cytokine kit (Linco Research). Resistin stimulated the release of IL-6 (Fig. 5A), IL-8 (Fig. 5B) and MCP-1 (Fig. 5C) from lung fibroblasts. Other cytokines analyzed were below the level of detection. These data suggest that resistin is able to initiate an inflammatory response in lung fibroblasts that may, in turn, contribute to the development and progression of fibrosis. The data suggest that resistin is a potent pro-inflammatory factor that is able to stimulate cytokine release from immune cells.

### Example 4

### Resistin Secretion is Upregulated by Pro-Fibrotic Mediators

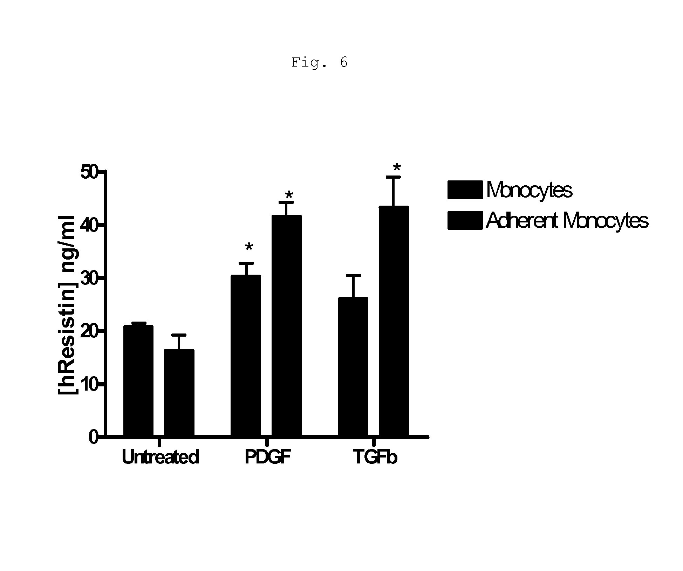

Human CD14⁺ monocytes purified from freshly isolated primary blood mononuclear cells were purchased from AllCells, LLC (Emeryville, CA). The cells were plated in 96 U-bottom well plate at a density of 75,000 cells per well in RPMI containing 10% fetal bovine serum (FBS). Cells were treated with 0 or 1 ng/ml TGFβ (R&D Systems, Minneapolis, MN) and PDGF (R&D Systems). After 48h of culture, resistin levels in the supernatants were measured by ELISA kit. To induce macrophage differentiation monocytes were plated in RPMI containing 10% FBS in 96 well flat-bottom plates and allowed to adhere, then the cells were treated similarly to described above. Data in Fig. 6 is expressed as mean ± S.E.M., n=3, * indicates p-value < 0.05.

As shown in Fig. 6, resistin is expressed and secreted from primary monocytes and adherent monocytes. TGFβ1 and PDGF treatment induced resistin production in both monocytes and adherent monocytes. Resistin was not detected in lung fibroblasts or other immune cells including T- or B-cells (data not shown). These data indicate that growth factors including TGFβ1 and PDGF that are present at sites of remodeling can induce the production of resistin from infiltrating monocytes and macrophages.

### Example 5

### Resistin is Expressed in Macrophage Cells in Lung

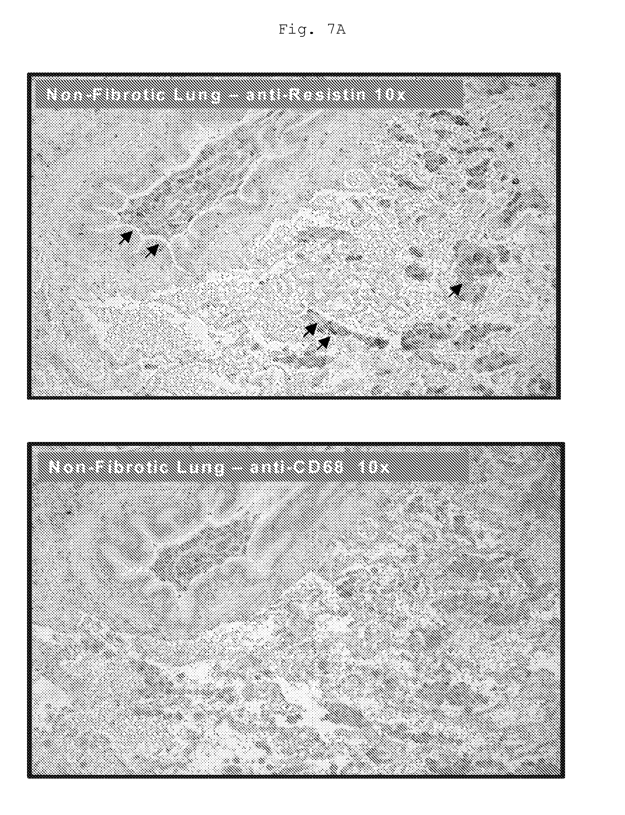

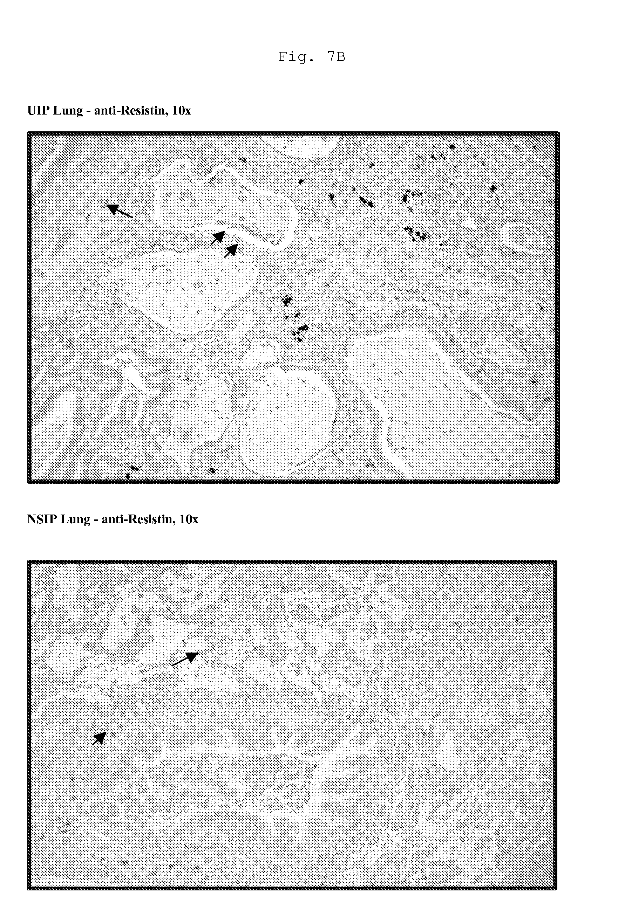

Lung biopsy samples from either the normal margins of lung tumors, UIP or NSIP patients (total n=9; n=3 of each patient cohort) were stained with an anti-resistin antibody (R&D Systems) at concentration 2µg/ml. In lung specimens, resistin protein was detected in macrophages, histiocytes (tissue-restricted macrophages) and mast cells. Co-staining of non-fibrotic lung specimen with high degree of inflammation with CD68 antibody showed that resistin co-localized with CD68-positive macrophage cells (Fig 7A). Expression of human resistin in monocytes and macrophage cells has been previously reported (Jung et al., Cardiovasc. Res., 69:76-85 (2006); Lehrke et al., PLoS Med., 1:e45 (2004)) as well as confirmed by our finding (Fig. 6). A representative example of staining with anti-resistin antibody of lung specimens from UIP and NSIP patients is shown in Fig. 7B. No clear difference in resistin staining was observed between non-fibrotic and fibrotic lung specimens.

### Example 6

### Modeling of Human Resistin 3-Dimensional Structure

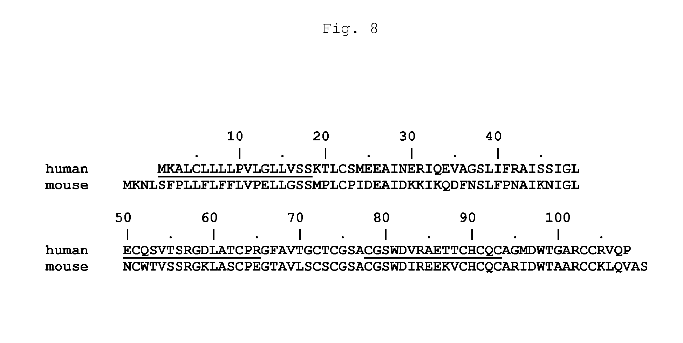

Amino acid sequences of human (SEQ ID NO: 1) and mouse resistin (SEQ ID NO: 2) were obtained from Swiss-Prot under the accession codes Q9HD89 and Q99P87, respectively and aligned. Fig. 8 shows the sequences alignment between human and mouse resistin; the overall sequence identity is 55%.

The crystal structure of mouse resistin (Patel *et al.*, *supra*) is available from the Protein Data Bank under the accession code 1RFX. Based on this structure, a 3D model of human resistin was constructed by replacing amino acids according to the sequence alignment. The computer program COOT (Emsley, Acta Crystallogr. D. Biol. Crystallogr. 60:2126-32 (2004)) was used for this purpose. The side chain conformation was adjusted to avoid close contacts with other residues. The most energetically favorable rotamers were chosen whenever possible. No changes in the main-chain conformation were necessary. The resulting model contained no residues in the forbidden conformation, no steric clashes, and no residues in an unfavorable environment, such as uncompensated charges in the interior of the molecule, or extensive hydrophobic patches on the surface of the molecule. Given that the sequence similarity to the mouse protein is relatively high and the stereochemical criteria are met, the resulting three-dimensional model provides a good approximation of the real structure.

The polypeptide chain of resistin is folded into an N-terminal α-helix (residues 22-47) followed by a globular domain (residues 48-108), which has a β-structure of a jelly-roll type. Three protein monomers form a trimer in which the α-helices form a coiled-coil, and the globular domains form a compact head with an extensive interface that covers about 30% of the surface of each monomer. An earlier theoretical model of resistin is available from the Protein Data Bank under accession code 1LV6. This earlier model is not in agreement with the crystal structure data now available for mouse resistin.

### Example 7

### Prediction of Hypothetical Resistin Receptor Binding Site

Several lines of evidence suggest that the side surface of the globular domains of the resistin trimer is a potential receptor-binding site. For example, resistin is active in a trimeric form (Patel *et al., surpa*), therefore only the surface accessible to solvent in the trimer is considered as a potential receptor-binding site. Further, as part of C1q/TNF structural superfamily of proteins, resistin may bind to its receptor in a way common to other family members that is at the side surface of the globular domain. The C1q/TNF superfamily is registered in the Pfam database under the accession code CL0100. It includes a number of cytokines, such as adiponectin, TNFα and β, and CD40L. Members of the superfamily have a characteristic helical/jelly-roll fold and a coiled-coil homotrimeric structure. Crystal structures are available for many members of the superfamily as well as for their complexes with the corresponding receptors. These structures indicate that receptor binding occurs at the side surface of the globular domains, usually at the domain interface, as observed for instance in APO2L+DR5 and TNFβ+TNFR55. Receptor was never observed being bound to the top of the globular domain or to the helical domain.

Also, the sequence similarity in the resistin family of proteins is much higher within the globular domain than in the helical domain suggesting its functional importance. Amino acid homology between human and mouse resistin is ∼36% in the helical domain versus ∼66% in the globular domain. Similarly, the homology between human and bovine or porcine resistin is ∼28% in the helical domain versus ∼85% in globular domain.

Lastly, the three-fold symmetry of the globular head of the resistin trimer implies that the receptor, if bound to the top of the head, should have an internal three-fold symmetry of its recognition site, which is very unlikely. This practically eliminates the possibility of binding to the top of the head.

There are only two fragments of the human resistin polypeptide chain that are on the side surface of the globular domain and that are solvent accessible in the trimeric form. These include residues 50-65 (SEQ ID NO: 3) and 78-93 (SEQ ID NO: 4). Both peptides contain two types of residues, those that are accessible and therefore may interact with the receptor, and those that are buried and therefore may not be involved in the interaction. Amino acids that are not on the surface might be replaced in order to achieve better biophysical properties of peptide. The following substitutions were made in the peptides to avoid non-specific disulfide cross-linking: C51S, C63S (peptide 1) and C78S, C89S, C91S, C93S (peptide 2) resulting in the following peptide epitope sequences:
Peptide 2815: ESQSVTSRGDLATSPR (SEQ ID NO: 5)
Peptide 2816: SGSWDVRAETTSHSQS (SEQ ID NO: 6)
Together, these two peptides cover 64% of the surface of the globular domain. An advantage of peptide 2815 is that all residues except the two cysteines are exposed in resistin, so that regardless of the folding of the peptide in the free form, the potential epitope residues will be available for antibody generation. Peptide 2816 forms a β-hairpin in the protein. Only one face of the β-hairpin may be involved in receptor recognition. The key residues here are R84-A85-E86. Another advantage of the peptide 2816 is that this region is 100% conserved in higher mammals and 75% conserved with respect to murine resistin. Given the high sequence similarity in this fragment, it is possible to raise an antibody that will cross-react with resistin protein from multiple species.

### Example 8

### Confirmation of functional importance of identified epitopes

*Generation of polyclonal antibodies:* The epitope peptides 2815 and 2816 were chemically synthesized on an ABI433A Peptide Synthesizer using RINK Resin SS (Advanced ChemTech, SA5030, Lot#17046). The peptide was cleaved from the resin, precipitated, filtered, washed and dried. Peptides were sent to Invitrogen Corporation (Carlsburg, CA) where they were fused to KLH protein for immunization. Two rabbits per peptide were immunized using the standard procedures (Cat. number M0300). ELISA assays on the peptide indicated antibody titers and the rabbits were terminally bled after the four-week immunization procedure. The antibody was affinity purified using peptide conjugated to serum albumin. The polyclonal antibody raised against the 2815 and 2816 peptides are referred to as C2815 and C2816, respectively.

*Binding to full length human resistin:* The ability of polyclonal antibodies C2815 and C2816 to bind full length human resistin was tested using a standard sandwich ELISA format. A plate was coated with 2 µg/ml C2815 or C2816 in 0.2M carbonate-bicarbonate buffer, pH 8.5 overnight at 4°C. To eliminate non-specific binding plates were blocked with 280 µl of 0.5% bovine serum albumin in PBS for 2 hours at 37°C. Human resistin at different concentrations was added to the plate and incubated overnight at 4°C. For detection biotin labeled anti-resistin antibody BAM1359 (R&D System, Minneapolis, MN) with europium was added followed by enhancement solution. Fluorescence signal was read on the Victor plate reader and data was plotted in GraphPad Prism.

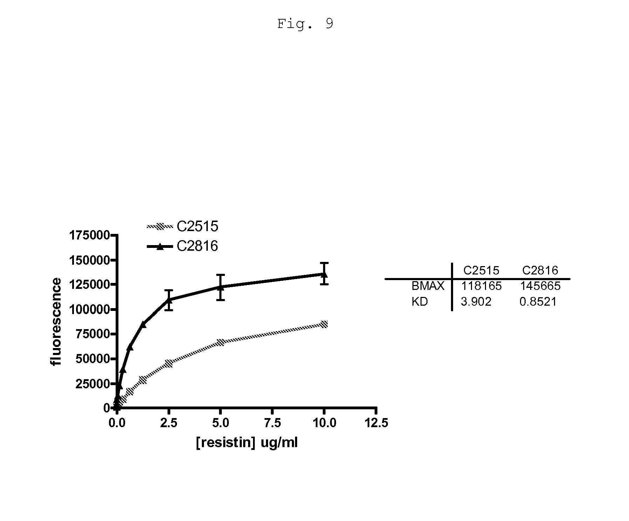

The results in Fig. 9 show binding of polyclonal antibodies C2815 and C2816 to full-length human resistin with a Kd of 3.9 and 0.8 µg/ml, respectively.

*Neutralizing activity:* The neutralizing activity of C2816 and C2815 was evaluated in a cytokine release assay. Briefly, 2ug/ml of human or mouse resistin was incubated with increasing concentrations of each antibody at room temperature for twenty minutes. The resistin-antibody mixture was added to isolated human primary blood mononuclear cells (PBMC) and incubated for twenty-four hours. Cell-free supernatants were collected and MCP-1 levels were detected by a commercially available ELISA kit (R&D System, Minneapolis, MN).

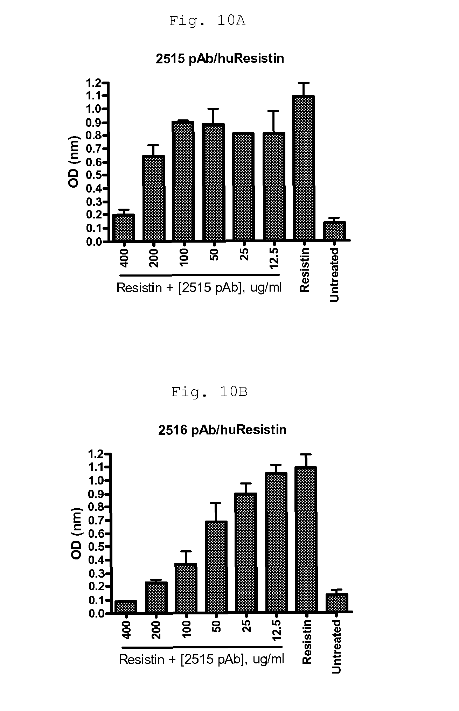

The results in Figs. 10A and B show the effect of polyclonal antibodies C2815 and C2816 on human resistin-mediated MCP-1 release from isolated human PBMC. The results indicate that the antibodies were able to inhibit dose-dependently the activity of human resistin in MCP-1 release assay confirming that identified epitopes are important for resistin activity. C2816 (IC50 65µg/ml) demonstrated superior neutralizing activity compared to C2515. However, that might be partially explained by higher affinity of C2816 to full-length human resistin protein (Fig. 9).

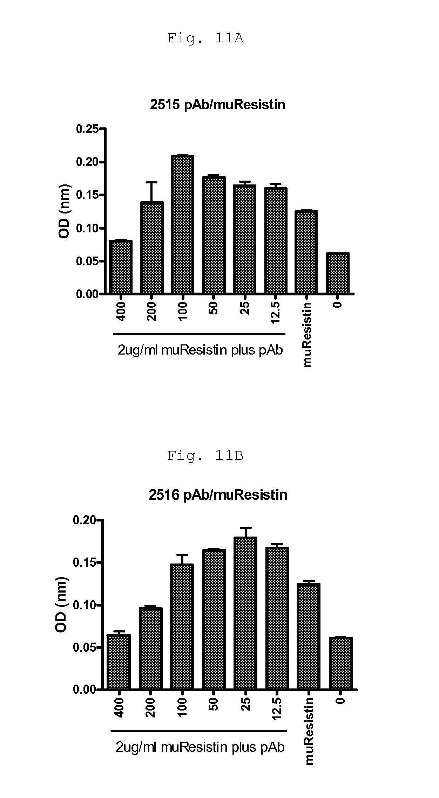

Figs. 11A and B show the effect of the C2815 and C2816 polyclonal antibodies on mouse resistin-mediated MCP-1 release from isolated human primary blood mononuclear cells. The results indicate that the antibodies were able to inhibit dose-dependently the activity of mouse resistin in MCP-1 release assay. The data suggest that C2815 and C2816 cross-react with mouse resistin in agreement with the high level of sequence conservation in the selected epitopes.

## Claims

1. An isolated antibody reactive with an epitope located at residues 78 to 93 defined by SEQ ID NO: 4 of human resistin.

2. A pharmaceutical composition comprising an effective amount of the resistin antagonist according to claim 1 and a pharmaceutically acceptable carrier or diluent.

3. The pharmaceutical composition of claim 2 for use in a method of modifying the biological activity of resistin in a mammal comprising administering the pharmaceutical composition to the mammal.

4. The pharmaceutical composition of claim 3 for use in a method of treating, or alleviating the symptoms of the diseases with aberrant fibroblast activity including interstitial lung diseases, hypertrophic scarring, keloid scarring, scleroderma, liver fibrosis, renal fibrosis and cardiac fibrosis comprising administering the pharmaceutical composition to a patient in need thereof.

## Patentansprüche

1. Isolierter Antikörper, reaktiv gegenüber einem an Resten 78 bis 93 gemäß SEQ ID NO: 4 lokalisierten Epitop von menschlichem Resistin.

2. Pharmazeutische Zusammensetzung, umfassend eine wirksame Menge des Resistin-Antagonisten gemäß Anspruch 1 und ein pharmazeutisch unbedenkliches Träger- oder Verdünnungsmittel.

3. Pharmazeutische Zusammensetzung nach Anspruch 2 zur Verwendung bei einem Verfahren zum Modifizieren der biologischen Aktivität von Resistin bei einem Säuger, umfassend Verabreichen der pharmazeutischen Zusammensetzung an den Säuger.

4. Pharmazeutische Zusammensetzung nach Anspruch 3 zur Verwendung bei einem Verfahren zum Behandeln oder Lindern der Symptome der Krankheiten mit anomaler Fibroblastenaktivität, einschließlich interstitiellen Lungenkrankheiten, hypertropher Narbenbildung, Keloidbildung, Sklerodermie, Leberfibrose, Nierenfibrose und Herzfibrose, umfassend Verabreichen der pharmazeutischen Zusammensetzung an einen diese benötigenden Patienten.

## Revendications

1. Anticorps isolé réactif avec un épitope localisé au niveau des résidus 78 à 93 définis par SEQ ID n° 4 de la résistine humaine.

2. Composition pharmaceutique comprenant une quantité efficace de l'antagoniste de résistine selon la revendication 1 et un véhicule ou diluant pharmaceutiquement acceptable.

3. Composition pharmaceutique selon la revendication 2, pour une utilisation dans une méthode de modification de l'activité biologique de la résistine chez un mammifère, comprenant l'administration de la composition pharmaceutique au mammifère.

4. Composition pharmaceutique selon la revendication 3, pour une utilisation dans une méthode de traitement ou de soulagement des symptômes des maladies présentant une activité aberrante des fibroblastes, comportant les maladies pulmonaires interstitielles, la cicatrisation hypertrophique, la cicatrisation chéloïdienne, la sclérodermie, la fibrose du foie, la fibrose rénale et la fibrose cardiaque, comprenant l'administration de la composition pharmaceutique à un patient en ayant besoin.
